# FASCICULE DE BREVET EUROPEEN

(11) **EP 1 778 297 B1**
(45) Date de publication et mention de la délivrance du brevet: **22.04.2009**
(21) Numéro de dépôt: 05797742.3
(22) Date de dépôt: 02.08.2005
(51) Int. Cl.: A61K 48/00, C12N 15/86

(54) **VECTEUR VIRAL ADENO-ASSOCIE POUR REALISER DU SAUT D'EXONS DANS UN GENE CODANT UNE PROTEINE A DOMAINES DISPENSABLES**
ADENO-ASSOZIIERTER VIRALER VEKTOR ZUR EXON-ÜBERSPRINGUNG IN EINEM FÜR EIN PROTEIN MIT ÜBERFLÜSSIGER DOMÄNE KODIERENDEN GEN
ADENO-ASSOCIATED VIRAL VECTOR FOR EXON SKIPPING IN A GENE ENCODING A DISPENSABLE-DOMAIN PROTEIN

(30) Priorité: 17.08.2004 FR 0451861
(43) Date de publication de la demande: 02.05.2007
(60) Demande divisionnaire: 08163844.7
(73) Titulaire: GENETHON, 91000 Evry (FR); CENTRE NATIONAL DE LA RECHERCHE SCIENTIFIQUE, 75794 Paris Cedex (FR)
(72) Inventeur: GARCIA, Luis, F-93200 SAINT DENIS (FR)
(74) Mandataire: Buchet, Anne
(86) Numéro de dépôt international: PCT/FR2005/050642
(87) Numéro de publication internationale: WO 2006/021724

(56) Documents cités:
- EP-A- 1 160 318
- WO-A-02/24906
- US-A1- 2004 040 052
- US-A1- 2004 101 514
- ANGELIS DE F G ET AL: "Chimeric snRNA molecules carrying antisense sequences against the splice junctions of exon 51 of the dystrophin pre-mRNA induce exon skipping and restoration of a dystrophin synthesis in Delta 48-50 DMD cells" PROCEEDINGS OF THE NATIONAL ACADEMY OF SCIENCES OF USA, NATIONAL ACADEMY OF SCIENCE. WASHINGTON, US, vol. 99, no. 14, 9 juillet 2002 (2002-07-09), pages 9456-9461, XP002250907 ISSN: 0027-8424
- WANG B ET AL: "Adeno-associated virus vector carrying human minidystrophin genes effectively ameliorates muscular dystrophy in mdx mouse model." PROCEEDINGS OF THE NATIONAL ACADEMY OF SCIENCES OF THE UNITED STATES OF AMERICA. 5 DEC 2000, vol. 97, no. 25, 5 décembre 2000 (2000-12-05), pages 13714-13719, XP002949073 ISSN: 0027-8424
- VAN DEUTEKOM J C ET AL: "Antisense-induced exon skipping restores dystrophin expression in DMD patient derived muscle cells." HUMAN MOLECULAR GENETICS. 15 JUL 2001, vol. 10, no. 15, 15 juillet 2001 (2001-07-15), pages 1547-1554, XP002250908 ISSN: 0964-6906
- BRUN C ET AL: "U7 SNRNAS INDUCE CORRECTION OF MUTATED DYSTROPHIN PRE-MRNA BY EXON SKIPPING" CMLS CELLULAR AND MOLECULAR LIFE SCIENCES, BIRKHAUSER VERLAG, BASEL, CH, vol. 60, no. 3, mars 2003 (2003-03), pages 557-566, XP009021235 ISSN: 1420-682X
- HOFFMAN E P ET AL: "Molecular pathophysiology and targeted therapeutics for muscular dystrophy" TRENDS IN PHARMACOLOGICAL SCIENCES, ELSEVIER, HAYWARTH, GB, vol. 22, no. 9, 1 septembre 2001 (2001-09-01), pages 465-470, XP004303319 ISSN: 0165-6147
- FLETCHER S ET AL: "Cryptic splicing involving the splice site mutation in the canine model of Duchenne muscular dystrophy." NEUROMUSCULAR DISORDERS : NMD. APR 2001, vol. 11, no. 3, avril 2001 (2001-04), pages 239-243, XP002363143 ISSN: 0960-8966
- KAPSA ROBERT ET AL: "Novel therapies for Duchenne muscular dystrophy." LANCET. NEUROLOGY. MAY 2003, vol. 2, no. 5, mai 2003 (2003-05), pages 299-310, XP002314368 ISSN: 1474-4422
- GREGOREVIC PAUL ET AL: "Systemic delivery of genes to striated muscles using adeno-associated viral vectors." NATURE MEDICINE. AUG 2004, vol. 10, no. 8, août 2004 (2004-08), pages 828-834, XP002314366 ISSN: 1078-8956
- BREMMER-BOUT MATTIE ET AL: "Targeted exon skipping in transgenic hDMD mice: A model for direct preclinical screening of human-specific antisense oligonucleotides." MOLECULAR THERAPY : THE JOURNAL OF THE AMERICAN SOCIETY OF GENE THERAPY. AUG 2004, vol. 10, no. 2, août 2004 (2004-08), pages 232-240, XP004552204 ISSN: 1525-0016
- GOYENVALLE AURÉLIE ET AL: "Rescue of dystrophic muscle through U7 snRNA-mediated exon skipping." SCIENCE. 3 DEC 2004, vol. 306, no. 5702, 3 décembre 2004 (2004-12-03), pages 1796-1799, XP002314369 ISSN: 1095-9203
- VACEK MARLA M ET AL: "High-level expression of hemoglobin A in human thalassemic erythroid progenitor cells following lentiviral vector delivery of an antisense snRNA." BLOOD, vol. 101, no. 1, 1 janvier 2003 (2003-01-01), pages 104-111, XP002376975 ISSN: 0006-4971
- LIU SONGKAI ET AL: "Inhibition of HIV-1 multiplication by antisense U7 snRNAs and siRNAs targeting cyclophilin A." NUCLEIC ACIDS RESEARCH. 2004, vol. 32, no. 12, 2004, pages 3752-3759, XP002376976 ISSN: 1362-4962
- KOBINGER GARY P ET AL: "Correction of the dystrophic phenotype by in vivo targeting of muscle progenitor cells." HUMAN GENE THERAPY, vol. 14, no. 15, 10 octobre 2003 (2003-10-10), pages 1441-1449, XP002376977 ISSN: 1043-0342
- LI S ET AL: "Stable transduction of myogenic cells with lentiviral vectors expressing a minidystrophin" GENE THERAPY, vol. 12, no. 14, juillet 2005 (2005-07), pages 1099-1108, XP002376978 ISSN: 0969-7128

## Description

La présente invention concerne l'utilisation de vecteurs viraux adéno-associés ou vecteur AAV, pour délivrer à des cellules cibles, des séquences antisens dirigées contre des sites d'épissage d'un gène codant une protéine à domaines dispensables, ainsi que ses applications thérapeutiques, en particulier dans le traitement de la maladie de Duchenne.

Ainsi, des séquences judicieusement choisies introduites dans un vecteur selon l'invention sont capables de donner naissance à des transcrits produisant une protéine dystrophine plus courte, mais fonctionnelle, corrigeant certaines formes de la maladie de Duchenne.

La dystrophie musculaire de Duchenne (DMD) est une maladie génétique portée par le chromosome X et qui touche environ 1 garçon sur 3 500. Elle se caractérise par l'absence d'une protéine de 427 kilodaltons, la dystrophine cytosquelettique, ce qui a pour conséquence la mort des fibres musculaires, corrélée à une détérioration musculaire sévère et progressive.

La dystrophine est une protéine modulaire présentant une région centrale composée de 24 domaines répétés de type "spectrin-like". Des protéines dépourvues de certaines de ces séquences répétées peuvent toutefois être parfaitement fonctionnelles ou du moins seulement partiellement défectives, comme constaté dans les formes atténuées (Becker) de DMD.

En revanche, la majorité des mutations graves du gène de la dystrophine consiste en des délétions d'un ou plusieurs exons perturbant le cadre de lecture du messager final ou bien des mutations ponctuelles, présentes dans les régions codantes ou exons, qui introduisent des codons stops ou des décalages de la phase de lecture. Dans les deux cas, ces mutations se traduisent par l'absence de dystrophine.

A titre d'illustration, un grand nombre de cas cliniques de la maladie de Duchenne est lié à des délétions multi-exoniques (génotypes DMD sévères : Δ45-50; Δ47-50 ; Δ48-50A 49-50 ; Δ50 ; Δ ) dont le cadre de lecture pourrait être rétabli par suppression de l'exon 51 (génotypes BMD légers: Δ45-51 ; Δ47-51 ; Δ48-514 49-51Δ 51-52).

Différentes stratégies et techniques ont été envisagées pour tenter de "réparer" les gènes mutés de la dystrophine.

Le remplacement du gène de la dystrophine dans les fibres musculaires malades, ou la compensation par des greffes de cellules saines des cellules nécrosées, a révélé des difficultés considérables.

La troisième voie envisagée, actuellement la plus exploitée, consiste à tenter de réparer l'ADN muté grâce à l'utilisation d'oligo-nucléotides antisens (ou AON) permettant de sauter certains exons et de parvenir ainsi à l'expression d'une protéine tronquée mais fonctionnellement efficace. Cette technique dite "saut d'exon" ou "exon skipping" préconise l'utilisation d'oligo-nucléotides complémentaires à des séquences impliquées dans l'épissage des exons à masquer.

Les études actuellement menées sur cette voie sont essentiellement réalisées sur des souris *mdx* atteintes de ladite maladie, en raison d'une mutation non-sens introduisant un codon stop dans l'exon 23 du gène murin de la dystrophine.

La difficulté principale de cette technologie consiste à introduire d'une manière stable et durable un oligo-nucléotide (AON) non dégradé dans les fibres musculaires malades, en particulier *in vivo.*

Dans un premier temps, il a été envisagé d'injecter directement lesdits oligonucléotides, éventuellement en association avec un détergent synthétique, tel que l'agent F127. Ainsi, des séquences avantageuses à administrer par cette voie pour masquer l'exon 19 ou 45 du gène humain de la dystrophine sont par exemple décrits dans les demandes de brevet EP 1 054 058 et EP 1 191 098, respectivement. Cependant, au vu de la courte durée de vie de ces oligonucléotides dans le muscle, ce mode de traitement nécessite des injections régulières, relativement contraignantes.

Par ailleurs, il a été tenté d'introduire ces séquences dans des vecteurs pour les véhiculer dans les cellules cibles. A ce jour, seuls des essais *in vitro* ont été réalisés : les constructions utilisées sont basées sur l'utilisation de rétrovirus et sont testées uniquement sur des cultures cellulaires. Les résultats rapportés se sont avérés peu probants, ou du moins insuffisants pour envisager une transposition *in vivo.*

Par exemple, le document WO 02/24906 illustre l'utilisation de ces deux méthodes distinctes pour exclure l'exon 46 dans des cellules humaines.

L'ensemble des travaux réalisés sur le transfert vectoriel des AON a principalement révélé que :
i) la présence de petites séquences de type snRNA (small nuclear RNA) permet une meilleure translocation au niveau du noyau des cellules cibles et une meilleure transcription des séquences. Ainsi, le document WO 03/095647 préconise la sélection avantageuse des snRNA de type U2 et U3 ;
ii) la présence simultanée de deux séquences cibles impliquées dans l'épissage d'un même exon améliore l'efficacité du saut dudit exon (1, 2).

Cependant, à cette heure, aucune solution pour réaliser du saut d'exons *in vivo,* hormis l'injection d'oligo-nucléotides avec les inconvénients mentionnés ci-dessus, n'a été proposée.

Pour la première fois, les inventeurs proposent une construction donnant des résultats *in vivo* remarquables quant à la restauration de l'activité de la protéine dystrophine dans le contexte de la maladie de Duchenne.

A cet effet, l'invention propose de construire un vecteur viral adéno-associé comprenant :
-une séquence snRNA modifiée de type U7;
- le promoteur natif de U7;
- au moins une séquence antisens dirigée contre au moins un site d'épissage d'au moins un exon, ledit exon codant un domaine dispensable de la dystrophine.

Dans la suite de la description, l'ensemble constitué par ces trois entités ,(séquence snRNA / promoteur U7 / séquence(s) antisens) est appelé "cassette U7".

Parmi la multitude de vecteurs disponibles, le Demandeur a avantageusement sélectionné un vecteur d'origine virale, à savoir un dérivé des virus adéno-associés ou AAV.

Parmi les 8 sérotypes identifiés, l'AAV utilisé dans le contexte particulier de la DMD est préférentiellement un AAV1, c'est à dire qu'il est composé d'une capside du sérotype 1. En effet, l'AAV 1 s'avère transduire le plus efficacement les cellules musculaires.

En revanche, les séquences d'origine virale, en particulier les ITR, associées au transgène sont avantageusement issues de l'AAV2. Il en résulte que le vecteur viral adéno-associé final est, selon un mode avantageux de réalisation, un pseudotype 2/1.

Ledit vecteur contient en outre une séquence snRNA modifiée. Les snRNA- ou small nuclear RNA sont des ARN de petite taille, présents dans le noyau des cellules et impliqués dans certaines étapes de maturation des pré-ARNm. Ils sont appelés U1, U2 ... U10.

Parmi ces différents types de snRNA, celui de type U7 normalement impliqué dans la maturation des ARN prémessagers codant les histones est préférentiellement utilisé en tant que transporteur.

Le snRNA en question peut être aussi bien d'origine humaine que murine, dans la mesure où ces petites séquences sont hautement conservées entre les différentes espèces. Préférentiellement, le snRNA utilisé dans l'invention est celui de souris.

On entend par "snRNA modifié", un ARN dans lequel les séquences impliquées dans la fonction initiale du snRNA sont inactivées. Ces séquences peuvent également être modifiées de manière à augmenter le niveau d'expression dudit snRNA.

Par exemple, dans le cas de U7, la séquence du site de fixation de la "small nuclear ribonucléoprotéine" (ou Sm protéine) est modifiée de manière à inactiver la maturation des ARN prémessagers codant les histones et parallèlement à augmenter la concentration nucléaire en U7snRNA. Par ailleurs, la séquence de 18 nucléotides complémentaires au site 3' de maturation des ARN prémessagers codant les histones est remplacée par les séquences antisens d'intérêt.

En pratique, ces modifications peuvent être introduites au moyen de la mutagénèse dirigée par PCR.

Le gène snRNA ainsi modifié est ensuite cloné dans le vecteur AAV, préférentiellement entre ses deux séquences ITR.

La présente invention pourrait également être réalisé avec des séquences U1 ou U2, mais moyennant davantage de modifications et pour une efficacité moindre.

Un vecteur selon l'invention comporte également un promoteur permettant d'exprimer les séquences antisens à un niveau suffisant pour assurer leur activité biologique et thérapeutique. De nombreux promoteurs utilisables dans le contexte des AAV sont connus de l'homme du métier. Cependant, selon un mode privilégié de réalisation, l'expression des séquences antisens est placée sous le contrôle du promoteur natif du snRNA utilisé dans la construction. Dans le cas où le U7 est préféré, c'est donc le promoteur de U7 qui assure la transcription des séquences antisens.

Le vecteur selon l'invention comporte également au moins une séquence antisens dirigée contre au moins un site d'épissage d'au moins un exon, c'est à dire capable d'interférer avec l'épissage dudit exon. La séquence antisens est préférentiellement une séquence complémentaire à au moins une séquence choisie dans le groupe suivant : site d'épissage 5' (donneur) ; site d'épissage 3' (accepteur) ; séquence intronique BP (Branching Point) ; et éventuellement des régions internes riches en purine, plus spécifiquement des ESE (Exon-internal Splicing Enhancer).

Avantageusement, pour assurer l'exclusion d'un même exon, deux séquences antisens ayant des cibles distinctes, préférentiellement le site 5' donneur et la séquence BP, sont introduites dans un même vecteur recombinant selon l'invention.

Il peut également être associé dans une même construction des séquences antisens dirigées contre des sites d'épissage d'au moins deux exons distincts.

Alternativement, il est possible d'utiliser plusieurs constructions, chacune portant une séquence antisens distincte, lesdites séquences étant dirigées contre un ou plusieurs exons.

En pratique, lorsque plusieurs séquences antisens (dirigées contre le même exon ou plusieurs exons distincts) sont associées, les différents cas de figure suivants peuvent se présenter :
- les séquences antisens sont intégrées dans une même cassette U7, portée par un unique vecteur AAV ; ou
- les séquences antisens sont intégrées dans différentes cassettes U7, portées par un unique vecteur AAV ; ou
- les séquences antisens sont intégrées dans différentes cassettes U7, chacune portée par un vecteur AAV.

Dans le cadre de l'invention, les séquences antisens sont spécifiques des différents sites d'épissage des exons constituant le gène de la dystrophine, quelque soit son origine.

Le gène murin de la dystrophine présente un intérêt évident puisque la souris constitue un modèle animal expérimental de choix. Ainsi, une souris *mdx*, portant une mutation dans l'exon 23 du gène murin de la dystrophine et produisant une protéine tronquée inactive, présente les symptômes de la DMD. Dans ce contexte, les séquences antisens sont donc dirigées contre les séquences impliquées dans l'épissage de l'exon 23.

Plus particulièrement, un vecteur selon l'invention comporte une séquence SEQ ID 1 comprenant un gène U7snRNA modifié comme décrit ci-dessus et intégrant des séquences antisens dirigées contre le site 5' donneur (SEQ ID 2) et la séquence BP (SEQ ID 3) de l'exon 23 du gène murin de la dystrophine placées sous le contrôle du promoteur U7, introduit entre les 2 séquences ITR du vecteur AAV.

De manière très intéressante, la fonctionnalité de tels vecteurs a été validée par le Demandeur chez un animal de grande taille, à savoir le chien. En effet, il existe des chiens naturellement myopathes en raison d'une mutation dans le site accepteur d'épissage de l'intron 6 de sorte que la non-prise en compte de l'exon 7 dans l'ARNm final annule le cadre de lecture, les exons 6 et 8 n'étant pas en phase. Le saut simultané des exons 6 et 8 permet théoriquement de restaurer un cadre de lecture opérationnel, l'ARNm final étant alors dépourvu des exons 6, 7 et 8. Ainsi, l'association de vecteurs selon l'invention, comprenant les séquences antisens SEQ ID 27 et 28, dirigées contre des régions ESE des exons 6 et 8, respectivement, s'est avérée efficace.

En vue de la thérapie humaine, les séquences antisens choisies sont dirigées contre au moins un site d'épissage d'au moins un exon du gène humain de la dystrophine, dont l'exclusion génère une protéine tronquée mais active.

Comme discuté précédemment, l'exon 51 du gène humain de la dystrophine, et plus particulièrement les séquences impliquées dans son épissage, sont des cibles avantageuses dans le cadre de l'invention. Ainsi, son exclusion du transcrit codant la dystrophine pourrait être bénéfique dans le traitement d'environ 20% des cas cliniques génotypés de maladie de Duchenne aujourd'hui répertoriés.

Ainsi, il a été montré par les inventeurs qu'une construction adaptée dans le cadre de l'invention comprenait la séquence SEQ ID 4, associant deux séquences antisens SEQ ID 5 et SEQ ID 6 dirigées contre des régions internes riches en purines de l'exon 51 du gène humain de la dystrophine, à la place des séquences SEQ ID 2 et 3 dans la séquence SEQ ID 1.

D'autres séquences antisens humaines, utilisables dans le cadre de l'invention, sont les suivantes :
- DA5' de séquence SEQ ID 7 dirigée contre le site 5' de l'exon 51 ;
- DA3' de séquence SEQ ID 8 dirigée contre le site 3' de l'exon 51 ;
- G5' de séquence SEQ ID 9 dirigée contre le site 5' de l'exon 51 ;
- GBP de séquence SEQ ID 10 dirigée contre lé site BP de l'exon 51 ;
- ESE4, ESE16 et ESE28 de séquence SEQ ID 11, 12 et 13, respectivement, dirigées contre des sites de type ESE de l'exon 51;
- Ex51AONlong1 de séquence SEQ ID 25 ;
- Ex51AONlong2 de séquence SEQ ID 26.

Ces séquences sont préférentiellement associées en tandem dans un vecteur selon l'invention. Préférentiellement, DA5' (SEQ ID 7) et DA3' (SEQ ID 8) ou G5' (SEQ ID 9) et GBP (SEQ ID 10) sont associées.

Les séquences SEQ ID 25 et SBQ ID 26 correspondent aux séquences SEQ ID 5 et SEQ ID 6 plus longues. Elles sont préférentiellement associées. Etant donné leur grande taille, chacune est avantageusement intégrée à une cassette U7, portée soit par le même vecteur AAV, soit par deux vecteurs AAV distincts à utiliser en tandem.

Fait donc également partie de l'invention tout type de cellule transfectéé par le vecteur recombinant, et en particulier des cellules musculaires, en particulier de type fibres musculaires (myotubes), précurseurs musculaires (myoblastes) ou toute cellule capable de différenciation musculaire. Ne sont pas visées par la présente invention les cellules souches humaines embryonnaires et leur utilisation.

Un tissu musculaire ou un organisme non humain transfecté par ledit vecteur sont également compris dans l'étendue de la protection recherchée. Parmi les organismes non humains, les animaux - en particulier les souris - sont préférés.

La présente demande décrit pour la première fois un potentiel thérapeutique pour les vecteurs revendiqués. La présente invention se rapporte donc également à des compostions pharmaceutiques comprenant comme principe actif au moins un vecteur tel que défini dans la présente demande, ainsi que l'utilisation de ce vecteur comme médicament. En outre, comme mentionné ci-dessus à propos des vecteurs lentiviraux, des cellules transfectées peuvent également avoir un potentiel thérapeutique dans le cadre de greffes.

Une composition pharmaceutique selon l'invention contient le vecteur ou les cellules revendiqués; associés à un véhicule pharmaceutiquement acceptable et inerte.

Lorsque les vecteurs selon l'invention sont à injecter au niveau des muscles malades, la composition pharmaceutique se présente préférentiellement sous forme liquide. La concentration en vecteur, la quantité à injecter et la fréquence des injections sont déterminées aisément par l'homme du métier..

Au vu des effets remarquables observés *in vivo* sur la restauration de la dystrophine dans les fibres musculaires atteintes de DMD, l'utilisation du vecteur ou des cellules selon l'invention pour la préparation d'un médicament destiné au traitement de la maladie de Duchenne est également revendiquée.

Hormis le dysfonctionnement de la dystrophine associé à la maladie de Duchenne illustré ici, certaines myopathies sont par exemple liées à un dysfonctionnement de la protéine à domaines dispensables qu'est la dysferline et peuvent donc être traitées avec un vecteur selon l'invention.

Dans leur emploi *in vivo,* des vecteurs selon l'invention se sont avérés être stables, avoir une localisation subcellulaire spécifique et produire des quantités thérapeutiquement actives et permanentes d'antisens.

Plus généralement, la présente demande démontre le potentiel du système AAV-U7snRNA comme outil pour l'inactivation ou la modification d'ARNm chez les animaux.

L'invention et les avantages qui en découlent ressortiront mieux des exemples de réalisation suivants à l'appui des figures annexées.

**Figure 1** **:**
**(A)** Le diagramme du haut montre une illustration schématique de la structure de la dystrophine intacte (427 kDa). Elle est composée de plusieurs domaines : un domaine de liaison à l'actine (ABD) en N-terminal, un domaine central en bâtonnet constitué de 24 motifs répétés "spectrine-like" (R) ainsi que 4 segments charnières (H) capables de conférer de la flexibilité, et un domaine riche en cystéines (CR) qui fixe la β-dystroglycane et d'autres membres du complexe associé à la dystrophine, à proximité de l'extrémité C-terminale. Le diagramme du milieu représente l'ARNm de la dystrophine (environ 14 000 bases) constitué de 79 exons. Dans les souris *mdx*, le remplacement d'une cytosine par une thymidine dans l'exon 23 à la position 3185 de la séquence codante crée un codon STOP prématuré. Le diagramme du bas met en .évidence les séquence cibles au niveau de la séquence BP (branch point ; BP22 ; SEQ ID 3) en amont de l'exon 23 et en aval du site donneur d'épissage (SD23 ; SEQ ID 2) pour forcer la machinerie en charge de l'épissage à sauter l'exon muté tout en maintenant un cadre de lecture ouvert.
**(B)** Structure du vecteur AAV(U7-SD23/BP22). La cassette U7-SD23/BP22 est constituée du promoteur U7 (position -267 à +1, boîte hachurée), la séquence U7 snRNA modifiée (boîte grisée et séquence correspondante en dessous) et les séquences en aval de la position 116 (boîte ouverte). Cette cassette a été placée entre deux séquences inversées répétées terminales (ITR) de AAV2 (SEQ ID 1).

**Figure 2** **:**
**(A)** Détection du snRNA modifié U7-SD23/BP22 (a) et du snRNA U7 endogène (B) suite à l'injection intramusculaire du vecteur AAV. Des échantillons d'ARN totaux ont été analysés à 0, 15 et 30 jours (colonnes 1, 2 et 3, respectivement) par RT-PCR. Les produits correspondants de 60pb ont été visualisés sur gel d'agarose. Détection de l'ARNm de la dystrophine avec le saut de l'exon 23. Des échantillons d'ARN totaux ont été analysés à 0, 15 et 30 jours par RT-PCR nichée, en utilisant des paires de primers dans l'exon 20 et 26. La bande de 901 pb qui correspond à l'ARNm sans saut (*) est la seule espèce détectée au jour 0 (colonne 1), et est progressivement remplacée par un fragment de 688 pb (**) qui correspond à l'ARNm ayant perdu l'exon 23.
**(B)** Séquence en ADN de la bande de 688 pb après purification sur gel.
**(C)** Immunodétection des protéines totales extraites des muscles jambiers antérieurs, colorées avec des anticorps monoclonaux anti-dystrophine Dysl (les flèches indiquent la dystrophine entière de 427 kDa : colonne 1, *mdx,* non injectée ; colonne 2, *mdx*, 2 semaines après injection, colonne 3, *mdx,* 1 mois après injection ; colonne 4, C57B16). Chaque colonne a été chargée avec une quantité totale de protéine de 40 gg. Le même profil a été observé avec des anticorps Dys2 (résultats non montrés).

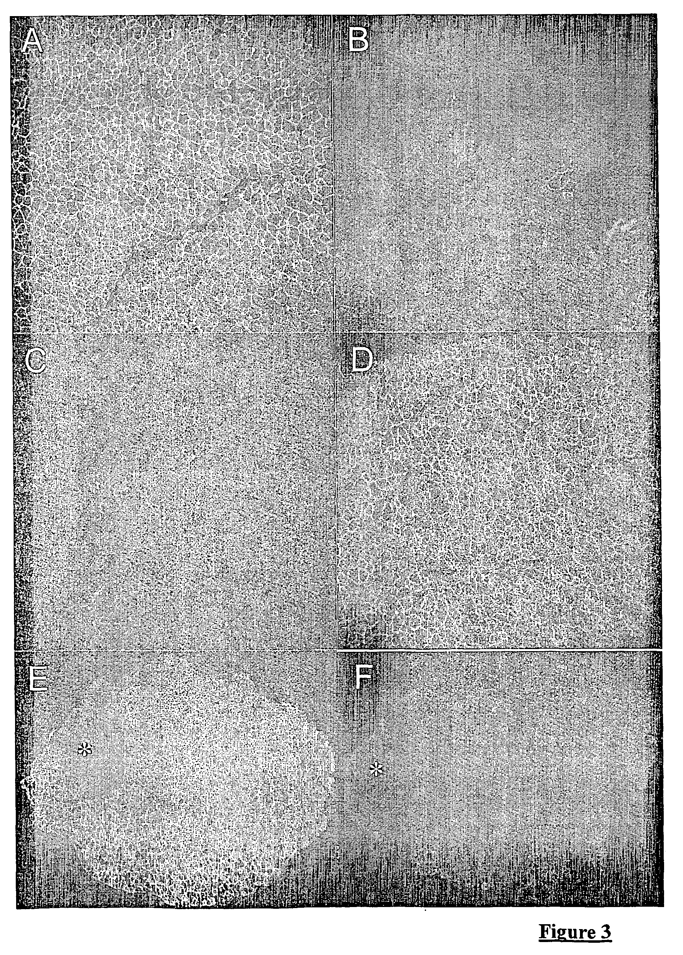

**Figure 3** : Restauration de la dystrophine dans des souris *mdx* après administration de AAV(U7-SD23/BP22). Coloration immunologique avec Dys2-Ab de sections transversales entières du "hind limb anterior" compartiment (muscle jambier antérieur = TA et extensor digitorum longus* = EDL) de **(A)** C57B 16 normale, **(B)** *mdx* non traitées, **(C-E)** souris *mdx* après 2, 4 et 12 semaines après injection intramusculaire, et **(F)** *mdx* 4 semaines après libération vectorielle intra-artérielle. Echelles (A-D) : 0,5 mm ; (E-F) : 1 mm.

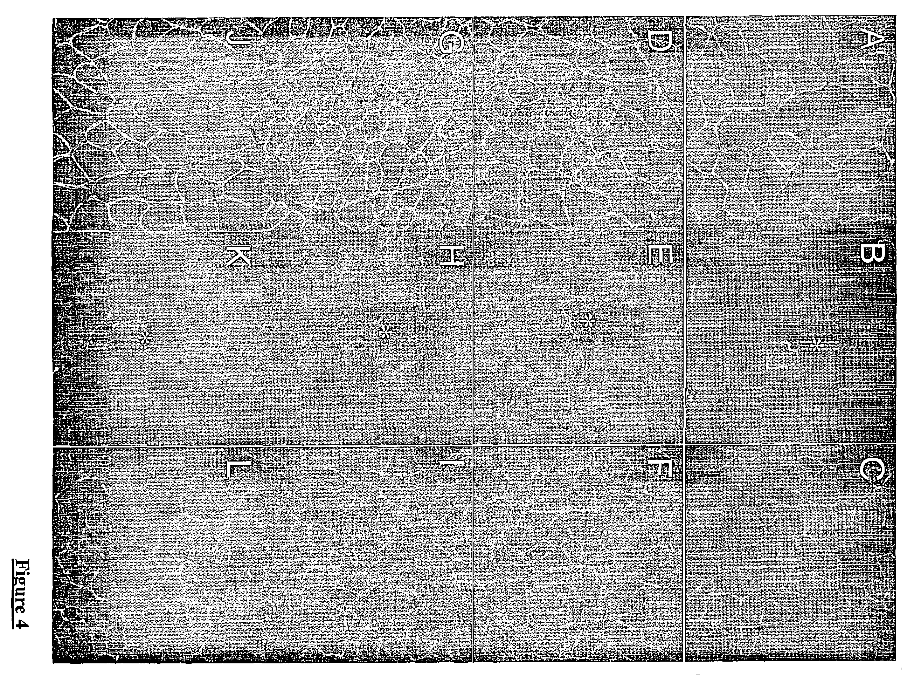

Figure 4 : Restauration du complexe protéique associé à la dystrophine dans des muscles *mdx* traités. Les colonnes de gauche, centrale et de droite montrent des sections du muscle jambier antérieur de C57B 16 normale, *mdx* non traitées et souris *mdx* après traitement, respectivement. Les sections ont été immunocolorées vis à vis de la dystrophine **(A,B,C),** de l'α-sarcoglycane **(D,E,F),** la β-sarcoglycane **(G,H,I)** et la β-dystroglycane **(J,K,L).** Le même lot de fibres révertantes (*), présentant de la dystrophine ainsi que le complexe protéique associé, est montré sur les séries de sections des souris non traitées.

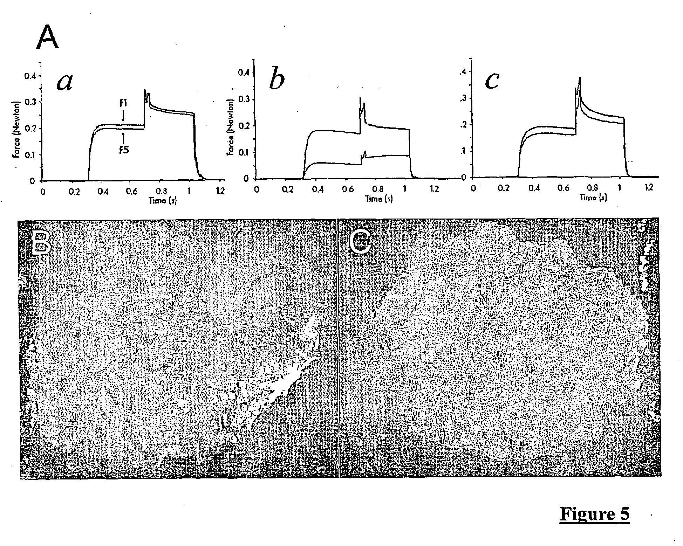

**Figure 5** **:** La restauration de la dystrophine dans des muscles *mdx* traités rétablit la susceptibilité normale au dommage induit par l'exercice. **(A)** enregitrements superposés de la tension produite par les muscles EDL de a) C57B16, b) *mdx* non traitées et c) souris *mdx*: après 45 jours de traitement, au cours de 5 contractions toniques avec extension forcée. Les muscles isolés ont été soumis à des stimulations répétitives (125 Hz) pendant 360 ms, à des intervalles de 3 min. Pendant les premières 160 ms, la tension s'est développée isométriquement, puis une extension forcée correspondant à 10% de la longueur L₀ pour laquelle le muscle produit une force maximale a été imposée à une vitesse de 1 longueur de fibre par seconde. Après relaxation, le muscle est revenu à sa longueur de repos. La chute de la force a été quantifiée par (F₁-F₅)/F₁, avec F₁ la force isométrique développée juste avant l'extension dans le premier tétanus, et F₅ celle de la cinquième. La chute de la force a atteint en moyenne 15% pour des muscles C57B16 contre 65% chez *mdx* Pour les *mdx* traités montrés en c), la réduction de la chute de la force a été de 17%, indiquant une pleine réacquisition des propriétés mécaniques des fibres musculaires. **(B** et **C)** Détection au bleu d'Evàns des fibres musculaires endommagées par l'exercice dans les jambiers antérieurs des pattes non traitées **(B)** ou traitées **(C)** d'un seul animal *mdx,* 60 jours après l'administration du traitement. Les fibres endommagées incorporent le bleu d'Evans, dont la fluorescence est détectée dans le canal rouge. La dystrophine a été colorée immunologiquement avec Ab-dys2 (vert).

**Figure 6** :
**(A)** Séquence de la région intron 5 - intron 8 chez le chien GMRD. La mutation responsable du phénotype est identifiée.
**(B)** Schéma de l'épissage de l'ARNm chez le chien GMRD.
**(C)** Schéma de l'interruption de la phase de lecture à la fin de l'exon 6 aboutissant à une dystrophine atrophiée.

**Figure 7** **:**
**(A)** Localisation des séquences cibles situées dans les séquences ESE de l'exon 6 (C6ESE2 ; SEQ ID 27) et de l'exon 8 (C8ESE1 ; SEQ ID 28).
**(B)** Schéma de l'épissage de l'ARNm chez le chien GMRD, après saut d'exons multiples réalisé avec les séquences antisens C6ESE2 et C8ESE1.
**(C)** Schéma de la séquence protéique de la dystrophine synthétisée après le saut d'exons multiples.

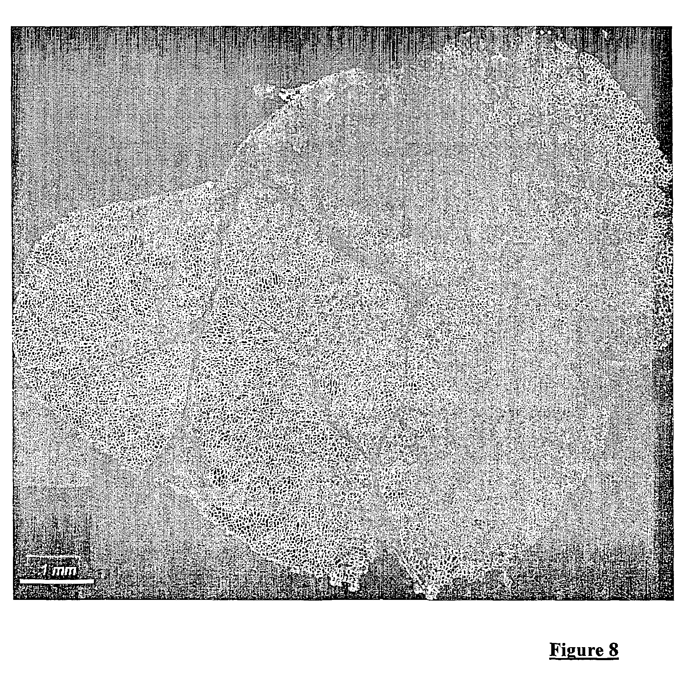

**Figure 8** **:** Restauration de la dystrophine dans des chiens GRMD adultes, obtenue 2 mois après une injection intramusculaire unique d'une préparation contenant les vecteurs AAV(U7-ex6) intégrant la séquence antisens SEQ ID 27 et AAV(U7-ex8) intégrant la séquence antisens SEQ ID 28. Coloration immunologique avec Dys2-Ab de sections transversales entières. Echelle 1 mm.

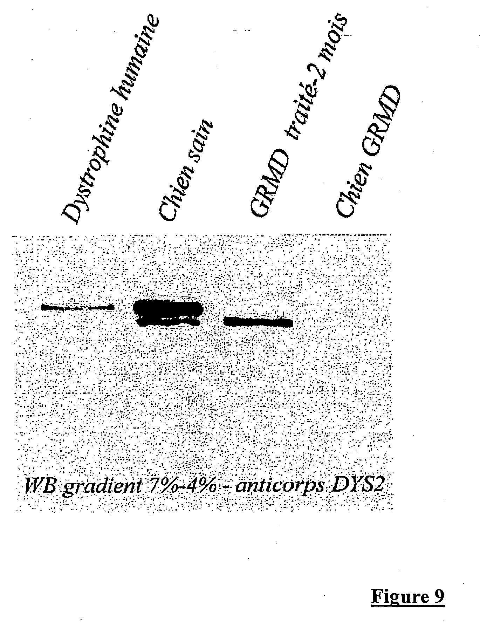

**Figure 9** **:** Immunodétection des protéines totales extraites des muscles jambiers antérieurs, colorées avec des anticorps monoclonaux anti-dystrophine Dys2 : colonne 1, dystrophine humaine chez sujet sain ; colonne 2, dystrophine chez chien sain, colonne 3, chien GRMD 2 mois après le traitement; colonne 4, chien GRMD non traité. Chaque colonne a été chargée avec une quantité totale de protéine de 40 µg.

**Fissure 10 :**
**(A)** Localisation des séquences cibles permettant le saut de l'exon 51 dans le gène humain de la dystrophine : la séquence antisens H51a a la séquence SEQ ID 6 et la séquence antisens H51 b a la séquence SEQ ID 5. Les séquences antisens AS et DS sont comme décrites par DeAngelis (**1**).
**(B)** Schéma de l'intégration de la cassette U7 dans le vecteur lentiviral.
**(C)** Détection de l'ARNm de la dystrophine humaine avec le saut de l'exon 51. Les échantillons d'ARN totaux ont été analysés par RT-PCR nichée. La flèche noire correspond à l'ARNm sans saut (*), alors que la flèche blanche indique l'ARNm dépourvu de l'exon 51.

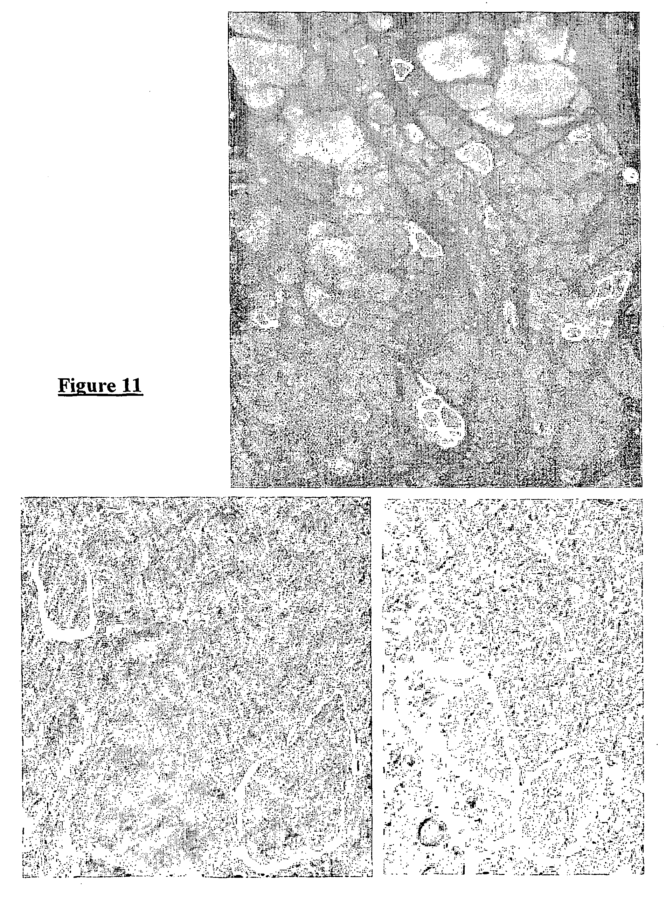

**Figure 11** **:** Restauration de la dystrophine dans des souris SCID-mdx, obtenue 1 mois et demi après l'injection dans les muscles tibialis antérieur de cellules souches delta 49-50 transduites par le vecteur Lent(U7-H51ab) intégrant les séquence antisens SEQ ID 5 et 6. Coloration immunologique avec Dys3.

### A- ETUDE EXPERIMENTALE CHEZ LA SOURIS

### I) MATÉRIEL ET MÉTHODES

### 1. Constructions

Le gène entier U7 snRNA (445 pb) a été obtenu par PCR sur l'ADN génomique de la souris avec les oligonucléotides: 5'-TAACAACATAGGAGCTGTG-3' (SEQ ID 14) et 5'-CAGATACGCGTTTCCTAGGA-3' (SEQ ID 15). Le domaine Sm (AATTTGTCTAG ; SEQ ID 16) a été optimisé en smOPT (AATTTTTGGAG ; SEQ ID 17), ainsi que décrit antérieurement (3), et la région du U7 capable de s'apparier avec le pré-ARNm a été échangée avec une séquence de 44 nucléotides complémentaires à la fois à la région couvrant la séquence BP (branch point) en amont de l'exon 23. du gène de la dystrophine (BP22: 5'-AAATAGAAGTTCATTTACACTAAC-3' ; SEQ ID 3) et la région en aval du site donneur d'épissage (SD23: 5'-GGCCAAACCTCGGCTTACCT-3' ; SEQ ID 2). Le fragment résultant U7smOPT-SD23/BP22 a ensuite été inséré entre 2 séquences inversées répétées terminales de AAV2 (SEQ ID 1).

### 2. Vecteurs

Des vecteurs recombinants pseudotypés AAV2/1 ont été préparés dans des cellules 293, comme déjà décrit **(4),** en cotransfectant 3 plasmides: pAAV2 (U7smOPT-SD23/BP22) codant le génome rAAV2, pXX6 arborant les fonctions "adenovirus helper" et pAAV1pITRC02 qui suppléent les gènes *rep* et *cap* de AAV1. Les titres en vecteurs ont varié entre 10¹² et 10¹³ génomes de vecteur (vg) ml⁻¹.

### 3. Animaux et méthodes de délivrance

Toutes les procédures animales ont été exécutées conformément au protocole approuvé par l'institution et dans des conditions strictes de confinement biologique. Un premier groupe de souris *mdx* (âgées de 8 semaines) a reçu par injection 50µl PBS (tampon salin phosphate) contenant 10¹² (vg) AAV(U7-SD23/BP22) dans les muscles jambiers antérieurs des pattes postérieures droites. Les muscles contre-latéraux ont été utilisés comme contrôles. Un second groupe de souris *mdx* du même âge ont été soumis à une perfusion intra-artérielle de 2x10⁻³ vecteurs à travers l'artère fémorale. Les souris ont été sacrifiées à des temps donnés, les muscles ont été congelés dans de l'isopentane refroidi à l'azote liquide et stockés à -80°C.

### 4. Histologie

Des coupes transversales (8 µm) en série, réalisées à intervalles de 200 µm sur la longueur du muscle, ont été examinées pour la dystrophine (NCL-DYS2; anticorps monoclonaux murins contre le domaine C-terminal; NovoCastra) et les protéines associées à la dystrophine β-dystroglycane, α et β-sarcoglycane; Novocastra) par détection immunologique, conformément aux instructions du fabricant. Les anticorps monoclonaux ont été détectés à l'aide d'anticorps biotinylés suivis par avidine-FITC (M.O.M Kit, Vector Laboratories). Les coupes préparées on été analysées par microscopie confocale à laser (Leica). Les tissus intermédiaires ont été collectés pour des analyses ultérieures de protéines et d'ARN.

### 5. Analyse par détection immunologique

Les sections des couches intermédiaires ont été collectées et extraites avec un tampon de lyse contenant 4% SDS, 125 mM Tris-HCl pH 6.4, 4 M urée, 10% β-mercaptoéthanol, 10% glycérol, 0.001 % bleu de bromophénol. Après séparation par centrifugation, le contenu en protéines a été mesuré à l'aide du test Bio-Rad "Protein Assay". Les échantillons, ajustés à 40 µg de protéines, ont été chargés sur des gels de polyacrylamide à 6%, soumis à électrophorèse et transférés sur des membranes de nitrocellulose qui ont été incubées avec soit NCL-DYS1 (anticorps monoclonaux murins contre la séquence répétée R8 du domaine "spectrine-like" en bâtonnet de la dystrophine; NovoCastra) ou NCL-DYS2, dilué au 1:100, suivi d'une incubation avec des anticorps secondaires de raifort conjugués à la peroxydase (1:1000) et un système d'analyse "ECL Analysis System" (Amersham).

### 6. Analyse d'ARN

Les ARN totaux ont été isolés d'un pool des coupes intermédiaires en utilisant le réactif TRIZOL (Life Technologies). Pour détecter U7 et U7smOPT-SD23/BP22, la transcription inverse a tout d'abord été réalisée sur les ARN totaux avec de la Supercript II reverse transcriptase en présence d'héxamères aléatoires (In vitrogen). Puis, les ADNc ont été amplifiés grâce à la Taq polymérase (Promega) avec 5'-AAGTGTTACAGCTCTTTTAG-3' (SEQ ID 18 localisée dans l'U7 sauvage) ou 5'-AAGGCCAAACCTCGGCTTAC-3' (SEQ ID 19 localisée dans l'U7smOPT-SD23/BP22) et 5'-AGGGGTTTTCCGACCGAAG-3' (SEQ ID 20) pour 30 cycles (94°C/30 s; 55°C/30 s; 72°C/30 s). Les produits de PCR ont été analysés sur des gels d'agarose à 2%. Pour détecter l'ARNm de la dystrophine, de la RT-PCR nichée a été réalisée avec 200 ng d'ARN totaux. La première réaction a eu lieu avec les primers Ex20ext (SEQ ID 21 ; 5'CAGAATTCTGCCAATTGCTGAG-3') et Ex26ext (SEQ ID 22 ; 5'-TTCTTCAGCTTGTGTCATCC-3') pendant 30 cycles (94°C/30 s; 55°C/1 min; 72°C/2 min). Puis 2 µl de la première réaction ont été amplifiés pendant 23 cycles avec Ex20int (SEQ ID 23 ; 5'-CCCAGTCTACCACCCTATCAGAGC-3') et Ex26int (SEQ ID 24 ; 5'-CCTGCCTTTAAGGCTTCCTT-3'). Les produits de PCR ont été analysés sur des gels d'agarose à 2%, et des bandes spécifiques ont été purifiées pour analyse de séquence.

### 7. Physiologie du muscle

Les muscles extensor digitorum longus (EDL) de souris contrôles ou traitées ont été disséqués pour évaluer les propriétés contractiles/mécaniques. Les muscles isolés ont été connectés d'un côté à un entraîneur électromagnétique et de l'autre côté à un capteur de force, et ont été stimulés au moyen d'électrodes placées parallèlement au muscle. Les contractions isométriques toniques et liées à une secousse brève (125 Hz; 360 ms, séparées par des temps de repos de 3 min) ont été étudiées à L₀ (la longueur à laquelle la force tonique isométrique maximale est observée). La tension isométrique a été calculée en divisant la force par la surface estimée de la section (cross-sectional area : CSA) du muscle. En supposant que les muscles ont une forme cylindrique et une densité de 1,06 mg.mm⁻³, la CSA correspond au poids humide du muscle divisé par sa longueur de fibres (5). Les contractions excentriques induisent d'une manière caractéristique des dommages musculaires corrélés à une rupture de membrane. Elles ont lieu lorsque un muscle contracté au maximum est étiré de force, ce qui mène à une chute de force. Ici, les muscles ont été allongés de 10% de la longueur L₀ pour laquelle le muscle produit une force maximale à une vitesse de 1 longueur de fibre par seconde. Cinq contractions excentriques ont été appliquées à des intervalles de 3 min. La chute cumulée de la force isométrique a été quantifiée comme décrit précédemment (5).

### II) RÉSULTATS

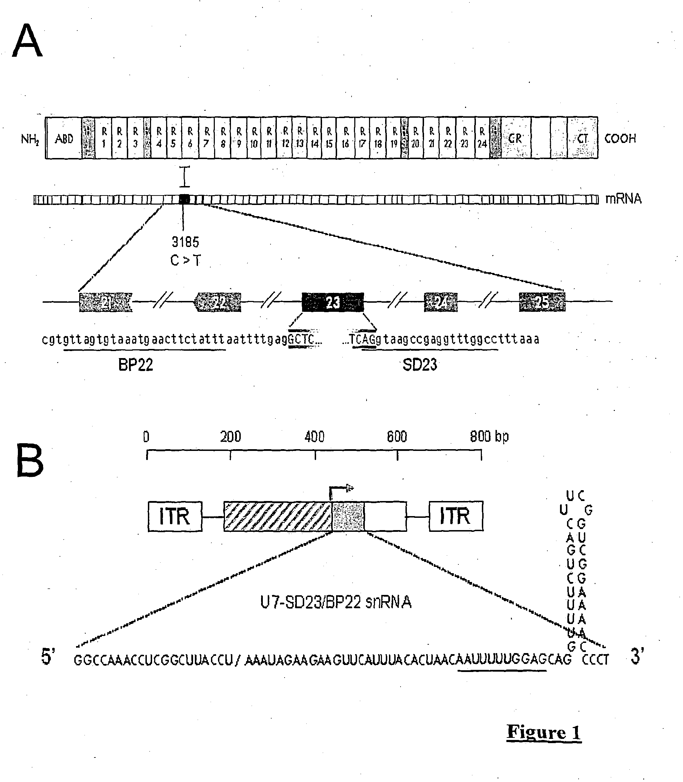

Un ARN de type U7 a été modifié afin d'y introduire des séquences antisens, aptes à interférer avec le processus de maturation des ARN messagers (ARNm), dans le noyau. La séquence du U7snRNA a été optimisée de manière à transporter des séquences antisens dirigées contre les introns 22 et 23 du gène murin de la dystrophine. Les séquences dans l'intron 22 ont été choisies de manière à entrer en compétition avec la fixation du U2snRNA au niveau du site d'épissage BP (Branching Point) (BP22 ; SEQ ID 3), et des séquences dans l'intron 23 correspondant au site de fixation de U1 au niveau du site donneur (SD23 ; SEQ ID 2) (Figure 1). Ces séquences ont été utilisées dans une stratégie de double cible, comme préconisé par Brun *et al.* **(2)**.

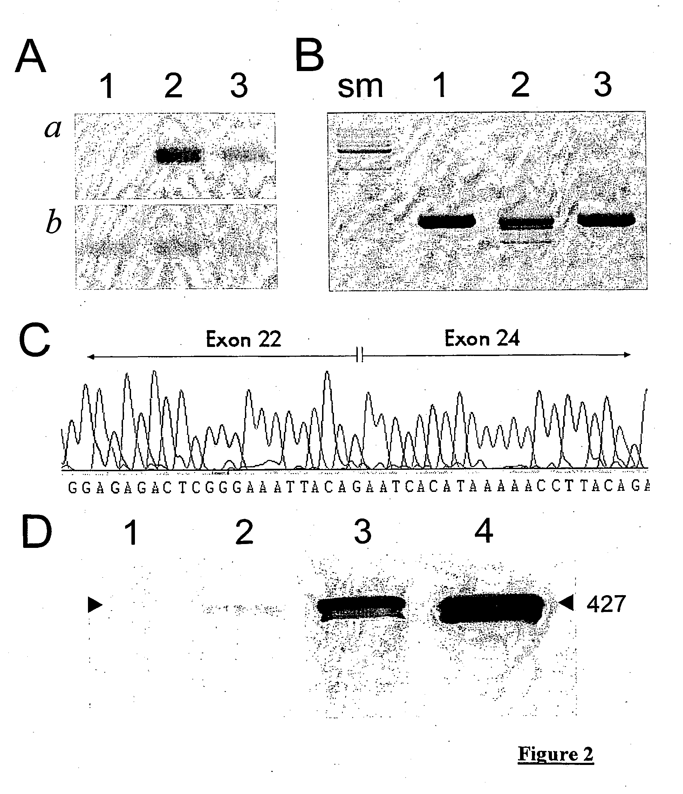

Le gène U7 modifié, comprenant à la fois le promoteur et les éléments 3', a été placé dans une construction basée sur AAV-2, qui a été introduite dans la capside AAV-1 pour obtenir une haute efficacité du transfert de gènes dans les muscles squelettiques. Des souris *mdx* adultes (âgées de 8 semaines) ont reçu en injection dans le muscle jambier antérieur une dose unique de 10¹⁰ génomes de vecteur et ont été analysées à différents temps, entre 2 et 16 semaines. L'analyse moléculaire du saut de l'exon 23 a été réalisée par RT-PCR nichée sur les ARN totaux préparés à partir des muscles ayant reçu l'injection. Un transcrit plus court, en l'occurrence dépourvu de l'exon 23, a été détecté. Il représentait de 5 à 10% du matériel amplifié, 2 semaines après injection et devenait l'espèce majoritaire après un mois (Figures 2B et 2C). Cette lente accumulation des transcrits ayant subi le saut d'exon n'a pas résulté de l'expression progressive du transgène pendant les premières semaines suivant le transfert de gènes médié par AAV, puisque les niveaux du U7 modifié, mesurés à 2 et 4 semaines, se sont avérés équivalents et représentaient 5 fois le niveau du U7snRNA endogène (Figure 2A). Plutôt, cela suggère une disponibilité limitée du pré-ARNm et une rotation lente de l'ARNm maturé de la dystrophine dans les fibres musculaires.

En accord avec la génération de transcrits ayant subi le saut d'exon, la protéine dystrophine a été détectée à la fois par immunodétection dans des extraits musculaires mais aussi par immunofluorescence sur des sections tissulaires (Figure 3). Les niveaux de dystrophine reflétaient ceux des ARNm produits (de 0,5% à 30% du niveau normal à 2 et 4 semaines, respectivement). Le saut d'exon a généré des espèces protéiques immunoréactives de la taille attendue, sans évidence de la présence de multiples produits de dégradation. Il est à noter que la différence en poids moléculaire entre la protéine sauvage et tronquée ne peut pas être résolue sur le gel montré. Virtuellement, toutes les fibres du muscle ayant reçu l'injection ont été détectées positivement à partir de 4 semaines après l'injection et la protéine était typiquement localisée à la périphérie des fibres. Des coupes histologiques des muscles traités ont montré distinctement la disparition du phénotype dystrophique, avec des fibres qui ont retrouvé leur forme polygonale normale et l'absence de cellules inflammatoires.

Un groupe d'animaux *mdx* a reçu le vecteur AAV-U7 par perfusion intra-artérielle à haute pression dans la patte inférieure. Cela a abouti, après un mois, à la restauration efficace de la dystrophine dans la plupart des fibres, dans tous les muscles de la patte perfusée, dont TA et EDL (Figure 3F). En plus de la dystrophine, les protéines associées incluant l'α-sarcoglycane, la β-sarcoglycanc et la β-dystroglycane ont été détectées à la périphérie des fibres des animaux traités (Figure 4). Cela a confirmé que le produit de l'ARNm ayant subi le saut d'exon contient un domaine C-terminal de fixation de la dystroglycane qui est essentiel à la fonction d'ancrage à la membrane de la dystrophine.

La susceptibilité aux dommages induits par l'exercice a été évaluée chez les animaux traités en mesurant la résistance aux contractions toniques suivies d'une élongation forcée. Pour cette expérience, les souris ont reçu une dose unique de AAV-U7 dans le muscle EDL et ont été analysées après 45 jours. Alors que les muscles des animaux *mdx* étaient incapables de supporter des élongations répétées, les muscles traités exprimant une dystrophine rétablie dans plus de 70% de leurs fibres présentaient des performances essentiellement équivalentes aux muscles normaux avec une chute de force de 17% suivant 5 stimuli, comparé à 15% pour les sauvages (Figure 5A). Les dommages induits par l'exercice ont aussi été évalués en soumettant les souris *mdx* injectées dans le muscle jambier antérieur à une course de grande envergure, suivie d'une injection au Bleu d'Evans, un colorant imperméable aux cellules. Les lésions musculaires, mises en évidence par l'entrée du colorant dans les fibres, étaient significatives dans le muscle traité (Figure 5B) et absentes dans le muscle non traité contre-latéral du même animal (Figure 5C).

### B- ETUDE IN VIVO CHEZ LE CHIEN

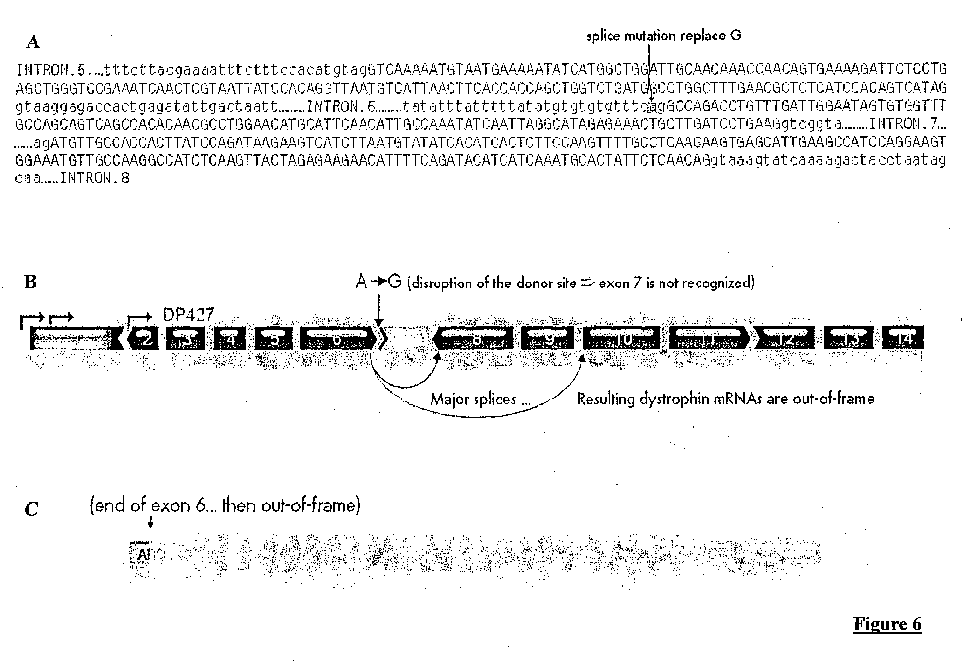

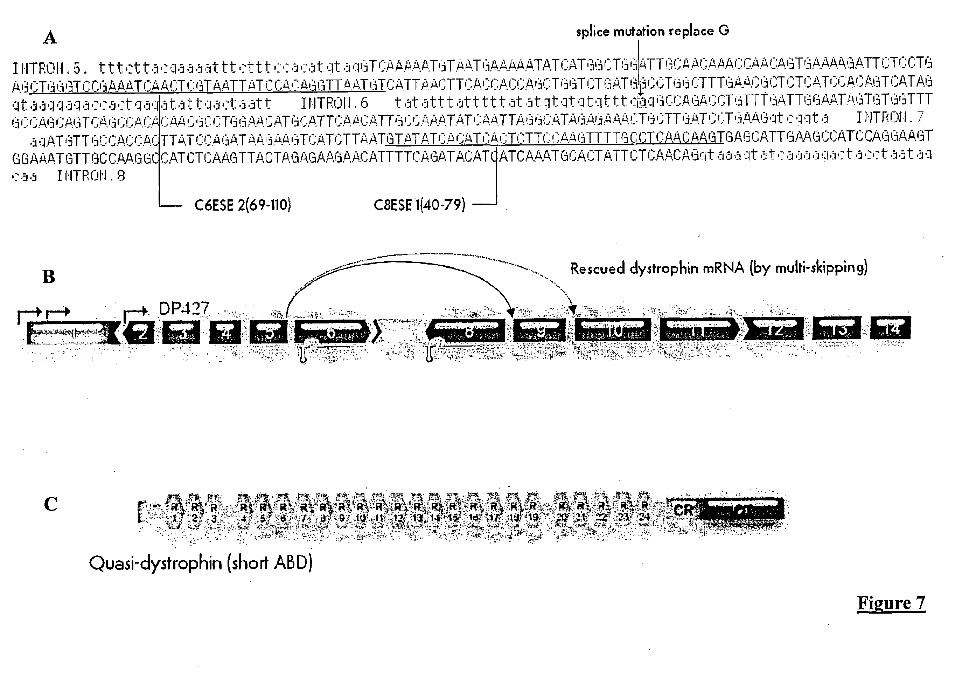

Les résultats obtenus chez la souris ont pu être transposés à un animal de grande taille, à savoir le chien GRMD. Le chien GRMD présente une mutation ponctuelle dans le site accepteur d'épissage de l'intron 6 (AG transformé en GG ; Fig. 6A), interdisant l'inclusion de l'exon 7 dans l'ARNm de la dystrophine (Fig. 6B). L'ARNm ainsi formé (delta exon 7) présente un décalage du cadre de lecture lié au déphasage des exons 6 et 8 (Fig. 6B et C).
Deux ARN de type U7 ont été modifiés afin d'y introduire des séquences antisens, aptes à interférer avec la machinerie d'épissage au voisinage des exons 6 et 8 (Fig. 7A), de sorte que les exons 6, 7 et 8 ne soient pas pris en compte dans l'ARNm final (multi-skipping ; Fig. 7B). Cet ARNm, dont le cadre de lecture est restauré, permet la production d'une protéine légèrement tronquée dans le domaine ABD (Fig. 7C), mais théoriquement parfaitement fonctionnelle.
L'efficacité des vecteurs AAV(U7-ex6) intégrant la séquence antisens SEQ ID 27 et AAV(U7-ex8) intégrant la séquence antisens SEQ ID 28 a été testée *in vivo* par injection loco-régionale chez des sujets GRMD adultes. La figure 8 illustre le niveau de dystrophine obtenu 2 mois après une injection intramusculaire unique d'une préparation de 500 µl contenant un mélange des deux vecteurs (~10¹¹ particules virales). Cette dystrophine est également détectable par Western-blot (figure 9). Comme chez la souris, le complexe de protéines associées à la dystrophine est également restauré suggérant indirectement le bon fonctionnement de la « quasi-dystrophine » induite par saut multi-exon.

### C- ETUDE IN VITRO CHEZ L'HOMME

Nous avons développé une approche permettant de combiner les approches de thérapie cellulaire et de saut d'exon dans des populations de cellules capables de participer à la régénération musculaire *in vivo.* Pour cela, nous avons développé des vecteurs lentiviraux véhiculant des cassettes U7 susceptibles d'induire des sauts thérapeutiques (modèles murin, canin et humain).

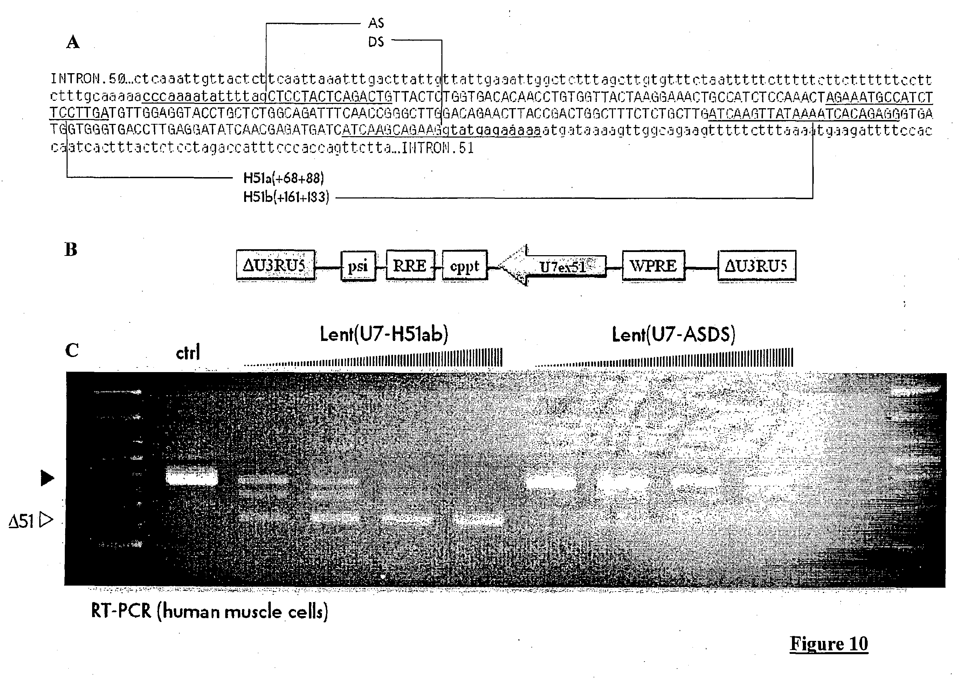

A titre d'exemple, nous présentons ci-dessous la restauration de dystrophine dans des cellules d'un patient DMD présentant une délétion des exons 49-50.
Différents vecteurs Lenti(U7ex51 humains) ont été générés, l'un d'entre eux portant les séquences précédemment décrites par DeAngelis (1) (Fig. 10A et B). Nos résultats montrent dans des conditions de transduction, de culture et d'analyse identiques que les séquences cibles précédemment publiée (U7-ASDS) ne fonctionnent que très modestement et ne permettent pas la restitution d'un niveau de dystrophine compatible avec un rationnel thérapeutique (Fig. 10C). En revanche, le vecteur (U7-H51ab), intégrant les séquences antisens SEQ ID 5 et SEQ ID 6, se révèle extrêmement efficace et autorise la modification quasi complète des ARNm dystrophine qui sont ici tous débarrassés de l'exon 51 ciblé par le vecteur (Fig. 10C). Des cellules souches AC133+ circulantes (20 x 10⁴) fraîchement prélevées chez un patient DMD (delta 49-50) ont été transduites par le vecteur Lent(U7-H51ab) intégrant les séquences antisens SEQ ID 5 et SEQ ID 6, et rapidement réinjectées dans les muscles tibialis antérieur de souris SCID-mdx dans le dessein de faire la preuve de la possibilité d'un saut d'exon thérapeutique *in vivo* à partir de cellules de patients DMD.
L'analyse histologique, après un mois et demi, démontre de façon conclusive la restauration de dystrophine humaine (révélation par anticorps DYS3 ne croisant pas avec la dystrophine murine) dans de nombreuses fibres musculaires vraisemblablement chimères homme/souris (Fig. 11).

### BIBLIOGRAPHIE

1) De Angelis FG, Sthandier O, Berarducci B, Toso S, Galluzzi G, Ricci E, Cossu G, Bozzoni 1, "Chimeric snRNA molecules carrying antisense sequences against the splice junctions of exon 51 of the dystrophin pre-mRNA induce exon skipping and restoration of a dystrophin synthesis in Delta 48-50 DMD cells", PNAS U.S.A. 2002 Jul 9;9(14):9456-61.
2) Brun C, Suter D, Pauli C, Dunant P, Lochmuller H, Burgunder JM, Schumperli D, Weis J, "U7 snRNAs induce correction of mutated dystrophin pre-mRNA by exon skipping ", Cell Mol Life Sci. 2003 Mar;60(3):557-66.
3) Gorman L, Suter D, Emerick V, Schumperli D, Kole R, "Stable alteration of pre-mRNA splicing patterns by modified U7 small nuclear RNAs", PNAS U.S.A. 1998; 95:4929-34.
4) Snyder RO et al.,- "Efficient and stable adeno-associated virus-mediated transduction in the skeletal muscle of adult immunocompetent mice", Hum Gene Ther 1997; 8: 1891-900.
5) Fougerousse F, Gonin P, Durand M, Richard 1, Raymackers JM, "Force impairment in calpain 3-deficient mice is not correlated with mechanical disruption ", Muscle Nerve 2003; 27: 616-23.

## Revendications

1. Vecteur viral adéno-associé comprenant :
- une séquence snRNA modifiée de type U7 ;
le promoteur natif de U7;
- au moins une séquence antisens dirigée contre au moins un site d'épissage d'au moins un exon, ledit exon codant un domaine dispensable de la dystrophine.

2. Vecteur selon la revendication 1 ***caractérisé* en ce que** le vecteur AAV est composé d'une capside du sérotype 1.

3. Vecteur selon La revendication 2 ***caractérisé* en ce que** le vecteur AAV est un pseudotype 2/1.

4. Vecteur selon d'une des revendications 1 à 3 ***caractérisé* en ce que** le site d'épissage est choisi dans le groupe comprenant le site 5' donneur, le site 3' accepteur, la séquence BP ("Branch Point") et les séquences ESE.

5. Vecteur selon d'une des revendications 1 à 4 ***caractérisé* en ce que** le vecteur comprend deux séquences antisens.

6. Vecteur selon la revendication 5 ***caractérisé* en ce que** les séquences antisens sont dirigées contre le site 5' donneur et la séquence BP, respectivement.

7. Vecteur selon la revendication 6 ***caractérisé* en ce que** les séquences antisens ont une séquence SEQ ID 2 et SEQ ID 3, respectivement.

8. Vecteur selon la revendication 7 ***caractérisé* en ce qu'**il comprend la séquence SEQ ID 1.

9. Vecteur selon la revendication 4, **caractérisé en ce qu'**il comprend une séquence antisens dirigée contre une séquence ESE de l'exon 6 et/ou de l'exon 8 du gène canin de la dystrophine, préférentiellement de séquence SEQ ID 27 et/ou SEQ ID 28.

10. Vecteur selon l'une des revendications 1 à 6 ***caractérisé* en ce qu'**au moins une séquence antisens est dirigée contre au moins un site d'épissage d'au moins un exon du gène humain de la dystrophine.

11. Vecteur selon la revendication 10 ***caractérisé* en ce qu'**au moins un exon est l'exon 51.

12. Vecteur selon la revendication 11 ***caractérisé* en ce que** les séquences antisens ont une séquence choisie dans le groupe comprenant les séquences SEQ ID 5, SEQ ID 6, SEQ ID 7, SEQ ID 8, SEQ ID 9, SEQ ID 10, SEQ ID 11, SEQ ID 12, SEQ ID 13, SEQ ID 25 et SEQ ID 26.

13. Vecteur selon la revendication 12 **caractérisé en ce qu'**il comprend les séquences SEQ ID 5 et SEQ ID 6, ou SEQ ID 7 et SEQ ID 8, ou SEQ ID 9 et SEQ ID 10, ou SEQ ID 25 et SEQ ID 26, respectivement.

14. Vecteur selon la revendication 13 ***caractérisé* en ce qu'**il comprend la séquence SEQ ID 4.

15. Vecteur selon l'une des revendications 1 à 5 et 9 à 10 **caractérisé en ce que** les séquences antisens sont dirigées contre des sites d'épissage d'au moins deux exons distincts.

16. Cellule isolée transfectée par un vecteur selon l'une des revendications 1 à 15, à l'exclusion d'une cellule souche humaine embryonnaire.

17. Cellule selon la revendication 16 ***caractérisée* en ce qu'**il s'agit d'une cellule musculaire.

18. Cellule selon la revendication 17 ***caractérisée* en ce qu'**il s'agit d'un myoblaste
ou d'une cellule capable de différenciation musculaire

19. Tissu musculaire isolé transfecté par un vecteur selon l'une des revendications 1 à 15.

20. Organisme non humain transfecté par un vecteur selon l'une des revendications 1 à 15.

21. Composition pharmaceutique comprenant un vecteur selon l'une des revendications 1 à 15 ou des cellules selon l'une des revendications 16 à 18.

22. Utilisation d'un vecteur selon l'une des revendications 1 à 15 ou de cellules selon l'une des revendications 16 à 18 pour la préparation d'un médicament destiné à traiter la maladie de Duchenne.

## Claims

1. Adeno-associated viral vector comprising:
- a U7 type modified snRNA sequence;
- the native U7 promoter;
- at least one antisense sequence directed against at least one splice site of at least one exon, the said exon encoding a dispensable domain of dystrophin.

2. Vector according to claim 1 ***characterised* by** the AAV vector being composed of a serotype 1 capsid.

3. Vector according to claim 2 ***characterised* by** the AAV vector being a 2/1 pseudotype.

4. Vector according to one of claims 1 to 3 ***characterised* by** the splice site being chosen from the group including the 5' donor site, the 3' acceptor site, the BP (Branch Point) sequence and the ESE sequences.

5. Vector according to one of claims 1 to 4 ***characterised* by** the vector containing two antisense sequences.

6. Vector according to claim 5 ***characterised* by** the antisense sequences being directed against the 5' donor site and the BP sequence, respectively.

7. Vector according to claim 6 ***characterised* by** the antisense sequences having an SEQ ID 2 and an SEQ ID 3 sequence respectively.

8. Vector according to claim 7 ***characterised* by** its containing the SEQ ID 1 sequence.

9. Vector according to claim 4, **characterised by** its containing an antisense sequence directed against an ESE sequence of exon 6 and/or exon 8 of the canine dystrophin gene, preferentially sequence SEQ ID 27 and/or SEQ ID 28.

10. Vector according to one of claims 1 to 6 ***characterised* by** at least one antisense sequence being directed against at least one splice site of at least one exon of the human dystrophin gene.

11. Vector according to claim 10 ***characterised* by** at least one exon being exon 51.

12. Vector according to claim 11 ***characterised* by** the antisense sequences having a sequence selected from the group comprising the sequences SEQ ID 5, SEQ ID 6, SEQ ID 7, SEQ ID 8, SEQ ID 9, SEQ ID 10, SEQ ID 11, SEQ ID 12, SEQ ID 13, SEQ ID 25 and SEQ ID 26.

13. Vector according to claim 12 ***characterised* by** its including the sequences SEQ ID 5 and SEQ ID 6, or SEQ ID 7 and SEQ ID 8, or SEQ ID 9 and SEQ ID 10, or SEQ ID 25 and SEQ ID 26, respectively.

14. Vector according to claim 13 ***characterised* by** its containing the sequence SEQ ID 4.

15. Vector according to one of claims 1 to 5 and 9 to 10 ***characterised* by** the antisense sequences being directed against splice sites of at least two distinct exons.

16. Isolated cell transfected by a vector according to one of claims 1 to 15, with the proviso that it is not a human embryonic stem cell.

17. Cell according to claim 16 ***characterised* by** its being a muscle cell.

18. Cell according to claim 17 ***characterised* by** its being a myoblast or a cell capable of muscle differentiation.

19. Isolated muscular tissue transfected by a vector according to one of claims 1 to 15.

20. Non-human organism transfected by a vector according to one of claims 1 to 15.

21. Pharmaceutical composition including a vector according to one of claims 1 to 15 or cells according to one of claims 16 to 18.

22. The use of a vector according to one of claims 1 to 15 or of cells according to one of claims 16 to 18 for the preparation of a medicinal product intended to treat Duchenne muscular dystrophy.

## Patentansprüche

1. Adeno-assoziierter Virusvektor, Folgendes aufweisend:
- eine modifizierte snRNA-Sequenz vom Typ U7;
- den nativen U7-Promotor;
- mindestens eine Antisense-Sequenz, die gegen mindestens eine Spleißstelle mindestens eines Exons gerichtet ist, wobei das Exon einen entbehrlichen Bereich des Dystrophins codiert.

2. Vektor nach Anspruch 1, ***dadurch gekennzeichnet, dass*** der AAV-Vektor aus einem Kapsid des Serotyps 1 besteht.

3. Vektor nach Anspruch 2, ***dadurch gekennzeichnet, dass*** der AAV-Vektor ein Pseudotyp 2/1 ist.

4. Vektor nach einem der Ansprüche 1 bis 3, ***dadurch gekennzeichnet, dass*** die Spleißstelle aus der Gruppe ausgewählt ist, die die 5'-Donorstelle, die 3'-Akzeptorstelle, die BP-Sequenz ("Branch Point") und die ESE-Sequenzen umfasst.

5. Vektor nach einem der Ansprüche 1 bis 4, ***dadurch gekennzeichnet, dass*** der Vektor zwei Antisense-Sequenzen umfasst.

6. Vektor nach Anspruch 5, ***dadurch gekennzeichnet, dass*** die Antisense-Sequenzen gegen die 5'-Donorstelle bzw. die BP-Sequenz gerichtet sind.

7. Vektor nach Anspruch 6, ***dadurch gekennzeichnet, dass*** die Antisense-Sequenzen eine Sequenz SEQ ID 2 bzw. SEQ ID 3 besitzen.

8. Vektor nach Anspruch 7, ***dadurch gekennzeichnet, dass*** er die Sequenz SEQ ID 1 umfasst.

9. Vektor nach Anspruch 4, **dadurch gekennzeichnet, dass** er eine Antisense-Sequenz, die gegen eine ESE-Sequenz des Exons 6 und/oder des Exons 8 des kaninen Gens des Dystrophins gerichtet ist, vorzugsweise der Sequenz SEQ ID 27 und/oder SEQ ID 28 umfasst.

10. Vektor nach einem der Ansprüche 1 bis 6, ***dadurch gekennzeichnet, dass*** mindestens eine Antisense-Sequenz gegen mindestens eine Spleißstelle mindestens eines Exons des humanen Gens des Dystrophins gerichtet ist.

11. Vektor nach Anspruch 10, ***dadurch gekennzeichnet, dass*** mindestens ein Exon das Exon 51 ist.

12. Vektor nach Anspruch 11, ***dadurch gekennzeichnet, dass*** die Antisense-Sequenzen eine Sequenz besitzen, die aus der Gruppe ausgewählt ist, die die Sequenzen SEQ ID 5, SEQ ID 6, SEQ ID 7, SEQ ID 8, SEQ ID 9, SEQ ID 10, SEQ ID 11, SEQ ID 12, SEQ ID 13, SEQ ID 25 und SEQ ID 26 umfasst.

13. Vektor nach Anspruch 12, **dadurch gekennzeichnet, dass** er jeweils die Sequenzen SEQ ID 5 und SEQ ID 6, oder SEQ ID 7 und SEQ ID 8, oder SEQ ID 9 und SEQ ID 10, oder SEQ ID 25 und SEQ ID 26 umfasst.

14. Vektor nach Anspruch 13, ***dadurch gekennzeichnet, dass*** er die Sequenz SEQ ID 4 umfasst.

15. Vektor nach einem der Ansprüche 1 bis 5 und 9 bis 10, **dadurch gekennzeichnet, dass** die Antisense-Sequenzen gegen Spleißstellen von mindestens zwei verschiedenen Exons gerichtet sind.

16. Isolierte Zelle, mit Ausnahme einer humanen embryonalen Stammzelle, die durch einen Vektor nach einem der Ansprüche 1 bis 15 transfiziert ist.

17. Zelle nach Anspruch 16, ***dadurch gekennzeichnet, dass*** es sich um eine Muskelzelle handelt.

18. Zelle nach Anspruch 17, ***dadurch gekennzeichnet, dass*** es sich um ein Myoblast oder eine Zelle handelt, die zur Muskeldifferenzierung in der Lage ist.

19. Isoliertes Muskelgewebe, das durch einen Vektor nach einem der Ansprüche 1 bis 15 transfiziert ist.

20. Nicht humaner Organismus, der durch einen Vektor nach einem der Ansprüche 1 bis 15 transfiziert ist.

21. Pharmazeutische Zusammensetzung, die einen Vektor nach einem der Ansprüche 1 bis 15 oder Zellen nach einem der Ansprüche 16 bis 18 umfasst.

22. Verwendung eines Vektor nach einem der Ansprüche 1 bis 15 oder von Zellen nach einem der Ansprüche 16 bis 18, zur Herstellung eines Medikaments, das zur Behandlung des Duchenne-Syndroms bestimmt ist.


### SEQUENCE LISTING

<110> GENETHON
   Centre National :de la. Recherche Scientifique
<120> VECTEUR VIRAL ADENO-ASSOCIE RECOMBINANT .POUR REALISER DU SAUT D' EXONS DANS UN GENE CODANT UNE PROTEINE A DOMAINES DISPENSABLES
<130> G143-B-21235 PCT
<150> FRG4.51861
   <151> 2004-08-17
<160> 28
<170> PatentIn version 3.1
<210> 1
   <211> 960
   <212> DNÀ
   <213> Artificial séquence
<220>
   <223> U7-SD23/BP22
<400>
   ttgggaaggg cgatcggtgc gggcctcttc gctattacgc cagctggcgc gctcgctcgc 60
   tcactgaggc cgcccgggca aagcccgggc gtcgggcgac ctttggtcgc ccggcctcag 120
   tgagcgagcg agcgcgcaga gagggagtgg ccaactccat cactaggggt tccttgtagt 180
   taatgattaa cccgccatgc tacttatcta cgtagccatg ctctagataa caacatagga 240
   gctgtgattg gctgttttca gccaatcagc actgactcat ttgcatagcc tttacaagcg 300
   gtcacaaact caagaaacga gcggttttaa tagtctttta gaatattgtt tatcgaaccg 360
   aataaggaac tgtgctttgt gattcacata tcagtggagg ggtgtggaaa tggcaccttg 420
   atctcaccct catcgaaagt ggagttgatg tccttccctg gctcgctaca gacgcacttc 480
   cgcaaggcca aacctcggct tacctaaata gaagttcatt tacactaaca atttttggag 540
   caggttttct gacttcggtc ggaaaacccc tcccaatttc actggtctac aatgaaagca 600
   aaacagttct cttccccgct ccccggtgtg tgagaggggc tttgatcctt ctctggtttc 660
   ctaggaaacg cgtatgtggc tagagcatgg ctacgtagat aagtagcatg gcgggttaat 720
   cattaactac aaggaacccc tagtgatgga gttggccact ccctctctgc gcgctcgctc 780
   gctcactgag gccgggcgac caaaggtcgc ccgacgcccg ggctttgccc gggcggcctc 840
   agtgagcgag cgagcgcgca gctgcattaa tgaatcggcc aacgcgcggg gagaggcggt 900
   ttgcgtattg ggcgctcttc cgcttcctcg ctcactgact cgctgcgctc ggtcgttcgg 960
<210> 2
   <211> 20
   <212> DNA
   <213> Artificial Sequence
<220>
   <223> SD23
<400> 2
   ggccaaacct cggcttacct 20
<210> 3
   <211> 24
   <212> DNA
   <213> Artificial sequence
<220>
   <223> BP22
<400> 3
   aaatagaagt tcatttacac taac 24
<210> 4
   <211> 468.
   <212> DNA
   <213> artificial séquence
<220>
   <223> U7-h51AON2/h51AON1
<400> 4
   gggtctagat aacaacatag gagctgtgat tggctgtttt cagccaatca gcactgactc 60
   atttgcatag cctttacaag cggtcacaaa ctcaagaaac gagcggtttt aatagtcttt 120
   tagaatattg tttatcgaac cgaataagga actgtgcttt gtgattcaca tatcagtgga 180
   ggggtgtgga aatggcacct tgatctcacc ctcatcgaaa gtggagttga tgtccttccc 240
   tggctcgcta cagacgcact tccgcaacct ctgtgatttt ataacttgat tcaaggaaga 300
   tggcatttct aatttttgga gcaggttttc tgacttcggt cggaaaaccc ctcccaattt 360
   cactggtcta caatgaaagc aaaacagttc tcttccccgc tccccggtgt gtgagagggg 420
   ctttgatcct tctctggttt cctaggaaac gcgtatgtgg ctagcttt 468
<210> 5
   <211> 23
   <212> DNA
   <213> artificial sequence
<220>
   <223> h51AON2
<400> 5
   cctctgtgat tttataactt gat 23
<210> 6
   <211> 20
   <212> DNA
   <213> artificial sequence
<220>
   <223> h51AON1
<400> 6
   tcaaggaaga tggcatttct 20
<210> 7
   <211> 24
   <212> DNA
   <213> artificial sequence
<220>
   <223> DA5'
<400> 7
   tttttctcat accttctgct tcat 24
<210> 8
   <211> 28
   <212> DNA
   <213> artificial sequence
<220>
   <223> DA3'
<400> 8
   gtctgagtag gagctaaaat attttggg 28
<210> 9
   <211> 20
   <212> DNA
   <213> artificial sequence
<220>
   <223> G5 '
<400> 9
   tatcattttt tctcatacct 20
<210> 10
   <211> 22
   <212> DNA
   <213> artificial sequence
<220>
   <223> GBP
<400> 10
   tttgggtttt tgcaaagaag ga 22
<210> 11
   <211> 26
   <212> DNA
   <213> artificial sequence
<220>
   <223> ESE4
<400> 11
   tgtcaccaga gtaacagtct gagtag 26
<210> 12
   <211> 25
   <212> DNA
   <213> artificial sequence
<220>
   <223> ESE16
<400> 12
   accacaggtt gtgtcaccag agtaa 25
<210> 13
   <211> 25
   <212> DNA
   <213> artifîcial sequence
<220>
   <223> ESE28
<400> 13
   gtttccttag taaccacagg ttgtg 25
<210> 14
   <211> 19
   <212> DNA
   <213> artificial sequence
<220>
   <223> U7-1 murin
<400> 14
   taacaacata ggagctgtg 19
<210> 15
   <211> 20:
   <212> DNA
   <213> artificial sequence
<220>
   <223> U7-2 murin
<400> 15
   cagatacgcg tttcctagga 20
<210> 16
   <211> 11
   <212> DNA
   <213> artificial sequence
<220>
   <223> Sm
<400> 16
   aatttgtcta g 11
<210> 17
   <211> 11
   <212> DNA
   <213> artificial sequence
<220>
   <223> smoPT
<400> 17
   aatttttgga g 11
<210> 18
   <211> 20
   <212> DNA
   <213> artificial sequence
<220>
   <223> primer U7
<400> 18
   aagtgttaca gctcttttag 20
<210> 19
   <211> 20
   <212> DNA
   <213> artificial sequence
<220>
   <223> primer u7 smOPT-5D23/BP22
<400> 19
   aaggccaaac ctcggcttac 20
<210> 20
   <211> 19
   <212> DNA
   <213> artificial sequence
<220>
   <223> primer commun u7
<400> 20
   aggggttttc cgaccgaag 19
<210> 21
   <211> 22
   <212> DNA
   <213> artificial séquence
<220>
   <223> Ex20ext
<400> 21
   cagaattctg ccaattgctg ag 22
<210> 22
   <211> 20
   <212> DNA
   <213> artificial sequence
<220>
   <223> Ex26ext
<400> 22
   ttcttcagct tgtgtcatcc 20
<210> 23
   <211> 24
   <212> DNA
   <213> artificial séquence
<220>
   <223> Ex20int
<400> 23
   cccagtctac caccctatca gagc 24
<210> 24
   <211> 20
   <212> DNA
   <213> artificial sequence
<220>
   <223> Ex26int
<400> 24
   cctgccttta aggcttcctt 20
<210> 25
   <211> 45
   <212> DNA
   <213> artificial sequence
<220>
   <223> ex51AON]ong1
<400> 25
   gcaggfacct ccaacatcaa ggaagatggc atttctagtt tggag 45
<210> 26
   <211> 45
   <212> DNA
   <213> artificial sequence
<220>
   <223> ex51AONlong2
<400> 26
   ccctctgtga ttttataact tgatcaagca gagaaagcca gtcgg 45
<210> 27
   <211> 41
   <212> DNA
   <213> artificial sequence
<220>
   <223> C6ESE2
<400> 27
   acattaacct gtggataatt acgagttgat ttcggaccca g 41
<210> 28
   <211> 39
   <212> DNA
   <213> artificial sequence
<220>
   <223> C8ESE1
<400> 28
   acttgttgag gcaaaacttg gaagagtgat gtgatatac 39